# EUROPEAN PATENT APPLICATION

(11) **EP 4 814 816 A1**
(43) Date of publication of application: **30.09.2026**
(21) Application number: 25166804.2
(22) Date of filing: 27.03.2025
(51) Int. Cl.: G06Q 10/04, A61B 5/00, G16H 40/63, G16H 40/67

(54) **VALIDATING SENSORS**

(71) Applicant: British Telecommunications Public Limited Company, London E1 8EE (GB)
(72) Inventor: The designation of the inventor has not yet been filed
(74) Representative: British Telecommunications public limited company Intellectual Property Department

(57) **Abstract**

A computer-implemented method of validating a sensor of a plurality of sensors monitoring the health of a subject. The method comprises obtaining sensor data and reference data for a first sensor of the plurality of sensors and comparing the sensor data with the reference data. A ticket, comprising an assigned status, is generated for the first sensor based on a result of the comparing. The assigned status is validated by comparing the sensor data to additional sensor data from a second sensor of the plurality of sensors. A certificate of the first sensor is updated with the ticket comprising the validated status of the first sensor.

## Description

### FIELD

The present disclosure relates to monitoring sensors, e.g. as part of a network of Internet of Things (IoT) devices. More specifically, the present disclosure relates to validating sensors based on their output data.

### BACKGROUND

The Internet of Things (IoT) is a technology framework that connects physical objects and devices to the Internet, allowing them to collect and exchange data for monitoring, control, and automation. IoT devices typically include computer systems embedded in, or attached to, home appliances and/or industrial appliances. For example, IoT devices may include, among other things, factory equipment, vehicles, environmental monitors, lighting equipment, laundry machines, and so on.

IoT ecosystems may include a multitude of IoT devices, each having their own unique specification.

### SUMMARY

There is provided a computer-implemented method of validating a sensor of a plurality of sensors monitoring the health of a subject, the method comprising:
obtaining sensor data and reference data for a first sensor of the plurality of sensors;
comparing the sensor data with the reference data;
generating a ticket, comprising an assigned status, for the first sensor based on a result of the comparing;
validating the assigned status by comparing the sensor data to additional sensor data from a second sensor of the plurality of sensors; and
updating a certificate of the first sensor with the ticket comprising the validated status of the first sensor.

The plurality of sensors may comprise: one or more personal sensors associated with the subject in an environment; and/or one or more ambient sensors associated with the environment.

The first and second sensors may be different types of sensors and the respective sensor data may be correlated.

Optionally, the validating is performed in response to the assigned status being indicative of the sensor data being anomalous based on the result of the comparing with the reference data.

The method may involve determining, based on the comparing of the sensor data with the reference data, whether the sensor data is anomalous due to sensor error. The validating may then be performed in response to determining that the sensor data is anomalous due not to sensor error. Optionally, the method comprises excluding, in response to determining that the sensor data is anomalous due to sensor error, further sensor data received from the first sensor.

The reference data may be based on a modelled normal operation of the first sensor. For example, the modelled normal operation is modelled based on inputs including one or more of: historic sensor data of the first sensor; historic tickets of the first sensor; and an electronic health record of the subject monitored by the first sensor.

The validating part of the method may comprise: changing the assigned status if the sensor data for the first sensor and the additional sensor data for the second sensor do not correspond within a predetermined tolerance based on the comparing; or maintaining the assigned status if the sensor data for the first sensor and the additional sensor data for the second sensor correspond within a predetermined tolerance based on the comparing.

The method may be iterated one or more times with a preset frequency. The preset frequency may be modified in response to the assigned status being indicative of the sensor data being anomalous based on the result of the comparing.

The validating part of the method may be performed based on a priority level of the first sensor relative to the plurality of sensors.

There is also provided a data processing system comprising a processor configured to perform the method.

There is also provided a computer program comprising instructions which, when the program is executed by a computer, cause the computer to carry out the method.

In general terms, a sensor validation process is provided which involves two checks: one against expected data for a given sensor; and another against sensor data of different (types of) sensors in the same environment. The second check may be done in dependence on the outcome of the first check, e.g. if the sensor data is in accordance with its own reference/modelled data then the validation is not necessary whereas if the sensor data is determined to be anomalous the validation with additional sensor data from one or more other sensors is done. In other versions, all sensor data is validated with the additional sensor data.

The process is done to validate the readings from the sensor so that it can be trusted, e.g. as part of a network of sensors monitoring the subject/environment. It provides a quick and confident picture of the overall status of all sensors used in an environment as per their operational condition, through certificates, so that any decision for intervention is weighed accurately and prioritised based on quality readings and not erroneous data.

### BRIEF DESCRIPTION OF THE FIGURES

Aspects of the present disclosure will now be described by way of example with reference to the accompanying figures, in which:
Figure 1 is a schematic illustration of a computer system suitable for implementing the methods described herein;
Figure 2A is a data processing workflow for validating sensors monitoring a subject and prioritising intervention, according to an embodiment, with data structures on the left representing different types of data or devices and corresponding analytical processes on the right;
Figure 2B shows an example sensor ticket and sensor certificate within the workflow of Figure 2A; and
Figures 3 to 5 are process flowcharts showing certain steps of respective methods according to embodiments.

### DETAILED DESCRIPTION

The present inventors have realised that, due to the scale of IoT ecosystems and the diversity of IoT devices, there is a substantial challenge in managing IoT devices. The complexity of IoT ecosystems (i.e., networks comprising IoT devices) arises from a multitude of IoT devices, each with their own unique specifications. This diversity makes it a challenge to ensure a standardized and consistent approach to device management as well as device anomaly detection and repair.

A typical IoT device certification, for example, may cover aspects like the hardware, software, safety testing, FCC, ISED, RED, operator acceptance, GCF, PTCRB, etc., of a given device. While this can help to provide a device that completes the service appropriately, it neglects to inform the current operational state of the device in its environment.

Accordingly, it is desired to provide means and methods to remotely detect and ascertain anomalies in IoT devices using adapted tickets and certificates for the devices/sensors.

For example, historical sensor data (optionally in combination with health records for the person or machine being monitored) will be utilised to influence a sensor's certificate grading. This grading will determine whether the sensor is in an anomalous state, dependent on a real-time updating of tickets. For example, tickets are used to determine whether a sensor's current state is normal using historical sensor data (e.g. including past tickets for the sensor as well as health records for the subject being monitored) to determine the grading of the ticket (e.g. based upon defined 'normal' data). The ticket will then influence the sensor's overall certificate. Finally, the sensor certificate may then be used, e.g. by a machine learning model, to determine the subject's intervention priority (e.g. machine repair, healthcare visit) compared to other subjects being monitored by other sensors. Overall, the dynamic validation mechanism presented enables more efficient prioritisation of intervention, e.g. industrial repair/maintenance or healthcare provision.

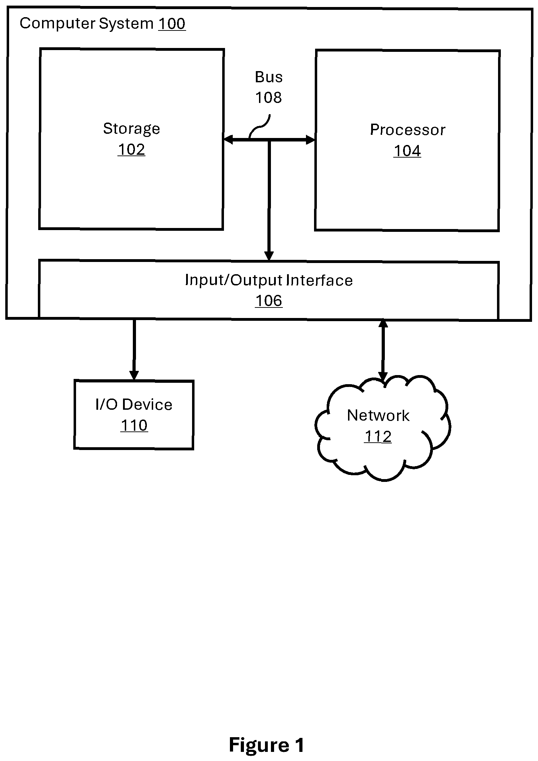

The relevant means and methods will now be described in detail with reference to Figures 1 to 5.

Figure 1 is a schematic illustration (or "block diagram") of a computer system 100 suitable for the operation of embodiments of the present invention. The system 100 comprises: a storage 102, a processor 104 and an input/output (I/O) interface 106, which are all communicatively linked over one or more communication buses 108.

The storage (or "storage medium" or "memory") 102 can include any volatile read/write storage device such as a random-access memory (RAM) and/or a non-volatile storage device such as a hard disk drive (HDD), a solid-state drive (SDD), magnetic disc, optical disc, read-only memory (ROM), flash memory, etc. The storage 102 can be formed as a hierarchy of a plurality of different storage devices, including both volatile and non-volatile storage devices, with the different storage devices in the hierarchy providing differing capacities and response times, as is well known in the art.

The processor 104 may include any known controller capable of executing computer-readable instructions, e.g. in the form of software or a computer program comprising such instructions. For example, the processor 104 comprises a microprocessor, microcontroller, central processing unit (CPU), graphics processing unit (GPU), or any other known processor. The computer-readable instructions may be stored in the storage 102. During operation of the system, the computer programs may be provided from the storage 102 to the processor 104 via the one or more buses 108 for execution. Alternatively, or additionally, the interface 106 can optionally comprise one or both of: a physical interface configured to receive a data carrier having such instructions stored thereon; and a receiver configured to receive a data carrier signal carrying such instructions. The instructions, when executed by the processor 104, cause the processor 104 to carry out a method according to an embodiment of the invention, as discussed below (and accordingly configure the system 100 to be a system 100 according to an embodiment of the invention).

The input/output (I/O) interface 106 provides interfaces to one or more devices 110 for the input or output of data, or for both the input and output of data. The one or more devices 110 may include user input interfaces (e.g., a keyboard or a mouse) which can receive inputs from a user of the computer system 100. The one or more devices 110 may additionally or alternatively include one or more user output devices (e.g. a display or a monitor) which can provide an output to the user of the computer system 100. Other such devices 110, such a touch screen monitor (not shown) may provide means for both inputting and outputting data.

The input/output (I/O) interface 106 may additionally or alternatively enable the computer system 100 to communicate with other computer systems via one or more networks 112. It will be appreciated that there are many different types of I/O interface 106 that may be used with computer system 100 and that, in some cases, computer system 100 may include more than one I/O interface. Furthermore, there are many different types of device 110 that may be used with computer system 100. The one or more devices 110 that interface with the computer system 100 may vary considerably depending on the nature of the computer system 100 and may include devices not explicitly mentioned above, as would be apparent to the skilled person. For example, in some cases, computer system 100 may be a server without any connected user input/output devices. Such a server may receive data via a network 112, carry out processing according to the received data and provide the results of the processing via a network 112.

It will be appreciated that the architecture of the system 100 illustrated in Figure 1 and described above is merely exemplary and that other computer systems 100 with different architectures (such as those having fewer components, additional components, and/or alternative components to those shown in Figure 1) may be used in embodiments of the invention. Examples of computer systems 100 that may be suitable for performing the methods described herein include, but are not limited to, IoT devices, personal computers (PCs), hand-held or laptop devices, multiprocessor systems, microprocessor-based systems, set top boxes, programmable consumer electronics, mobile telephones, smartphones, tablets, minicomputers, and/or network appliances (e.g., routers), or indeed any other computing device with sufficient computing resources to carry out a method according to embodiments of this invention. It will be appreciated that certain IoT devices may have one or more network interfaces (e.g. a receiver and transmitter) but may not have a user interface (e.g. the one or more user interface device 110).

Receivers and transmitters as described herein may be standalone or may be comprised in transceivers. A communication link as described herein comprises at least one transmitter capable of transmitting data to at least one receiver over one or more wired or wireless communication channels. Wired communication channels can be arranged for electrical or optical transmission. Such a communication link can optionally further comprise one or more relaying transceivers.

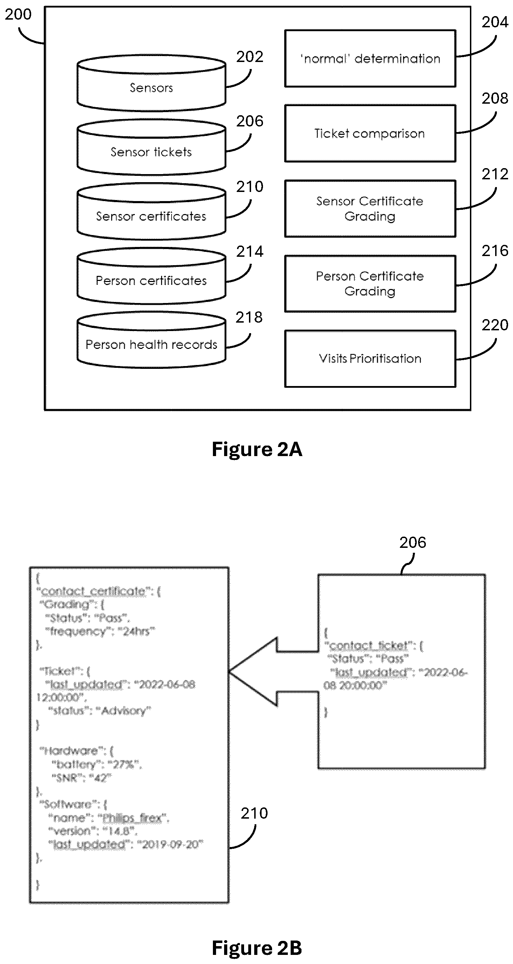

Referring now to Figure 2A, a data processing workflow 200 for validating sensors monitoring a subject and prioritising intervention is shown. It comprises sequential stages, with the components on the left representing different types of data storage and those on the right representing corresponding analytical processes.

In this specific embodiment, the sensors 202 are deployed to monitor a person in their home such that visits to the person can be prioritised against other persons being monitored.

The plurality of sensors 202 are part of a communication network. For example, multiple IoT devices comprising one or more respective sensors are connected as part of an IoT ecosystem within the environment, e.g. in this case the home. Thus, each sensor (or IoT device) may be communicatively coupled to one or more other sensors (or IoT devices) in the network (or IoTecosystem) by any suitable communication link. In some examples, there may be a mixture of IoT and non-loT devices in the network.

The sensors 202 are configured to collect data representative of measurements, e.g. of physical properties of the person or environmental conditions.

A normal data output 204 of a sensor (e.g. of an IoT device) monitoring the subject or environment can be determined, e.g. defined, using past data. For example, a PIR sensor may average 200 pings a day or a heartrate monitor may measure an average resting heartrate of 70 bpm. The normal data output of the sensor may be modelled, e.g. based on the past data. The modelled 'normal' may include a one or more threshold values which define an expected range of values for the sensor data.

An expected value or range of expected values that may be measured by the sensor (e.g. a data range indicative of normal or expected operation of the sensor) may thus be specified as reference data 204. Values measured by the sensor that are outside of the range specified in the reference data may be indicative of undesirable or anomalous operation of the sensor, for example, as described later.

A sensor (or device) ticket 206 is a record that tracks the status and performance of a sensor (or device). Figure 2B shows an example. It typically includes the unique identifier of the sensor 202 along with its current operational state (e.g. active, inactive, or malfunctioning), the date/time when the status was recorded, and the physical or logical location of the sensor 202 as part of the network. Additionally, it may contain any alerts or notifications related to the performance of the sensor 202, and/or any notes or observations. The information in the sensor tickets 206 helps in monitoring and maintaining the functionality of the respective sensors 202 and addressing any issues promptly.

A sensor (or device) certificate 210 is a digital record that compiles previous tickets 206 for a sensor (or device), providing a detailed history of its performance and status over time. It may also include the sensor's unique identifier, operational states recorded in past tickets, timestamps of these records, and/or the sensor's location. Figure 2B shows an example in which a sensor certificate 210 is being updated with a sensor ticket 206 for a contact sensor that has a timestamped 'pass' status. The certificate 210 in this example also includes a sensor certificate grading 212 and has a set frequency variable for checking the sensor status.

As well as including details about the sensor (e.g. its model number, manufacturer, specifications, etc.), the electronic certificate 210 can also be used for verifying characteristics of a given sensor 202, e.g. its calibration status, performance, and compliance with specific standards.

Additionally, or alternatively, the sensor certificate 210 may contain information about the software, such as firmware version and updates, as well as any relevant test results and performance metrics of the sensor 202. For example, the example certificate 210 of Figure 2B also includes information about the hardware (battery level and Signal-to-Noise Ratio, SNR) and software (name, version and last update) of the given sensor 202.

In general, the sensor certificates 210 allow for monitoring the long-term functionality of the sensors 202, ensuring they can be trusted for accurate and reliable measurements, and to help with addressing any recurring issues.

In a similar way to the sensor certificates 210 for the sensors 202, the workflow 200 for prioritising intervention optionally includes subject certificates 214 for the subjects, e.g. persons, being monitored. The subject (person) certificates 214 may be graded 216, e.g. with a status, in a similar way too.

An Electronic Health Record (EHR) 218 for the subject, e.g. person, may be another component of the workflow 200. In a medical context, the EHR comprises medical data about the subject that may be used by the methods and systems to carry out the validation and/or prioritisation. For example, the medical data may include physiological parameters of the subject, such as their age, weight and height. The medical data may additionally (or alternatively) include details of any medical conditions experienced by the subject as well as any medicine(s) that has been prescribed to treat those conditions.

In certain examples, a combination of EHRs with historical sensor data can be utilised to influence a sensors certificate grading 212. This grading will determine whether the sensor 202 is in a pass, fail or advisory state, dependent on a real-time updating of sensor tickets 206. Tickets are used to determine 204 whether a sensor's current state is anomalous (e.g. using historical sensor data as mentioned, plus historic tickets 206 and/or personal EHRs 218) to determine the grading of the ticket (based upon the defined 'normal' for the sensor monitoring that subject). The sensor ticket 206 will then influence the sensor's overall certificate 210. This sensor certificate 210 can then be used to determine a given subject's healthcare and wellbeing priority compared to other subjects, e.g. by a prioritisation model which may employ machine learning, to determine a visit prioritisation 220 among the respective subjects.

Overall, this system provides an efficient determination of the overall status of all sensors used in a subject's environment as per their operational condition, through sensor certificates, to ensure any decision for intervention (e.g. per the prioritisation model) is weighed accurately and prioritised based on quality readings and not erroneous data.

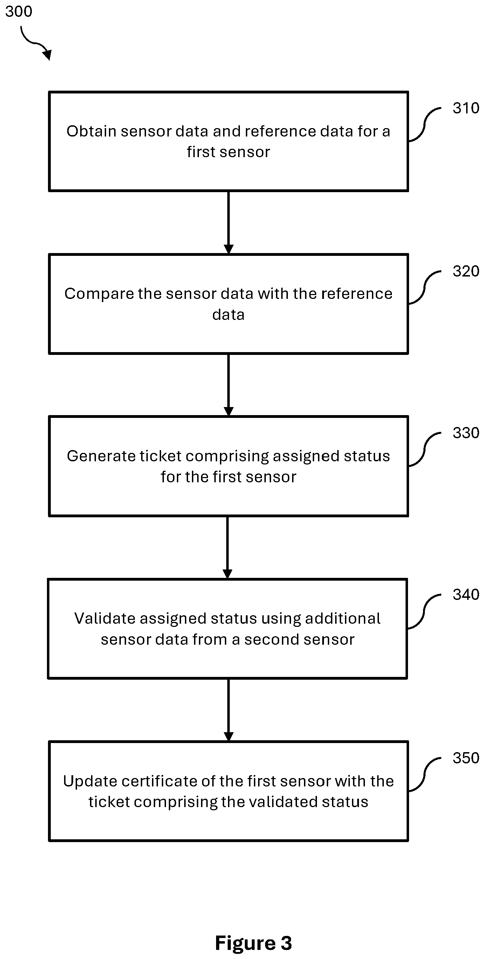

Figure 3 is a process flowchart showing certain steps of a computer-implemented method 300. For example, the method 300 is performed by a computer system 100, like the one described with reference to Figure 1, communicatively coupled to a plurality of sensors 202 via a network 112.

The plurality of sensors, for example, comprises one or more personal sensors associated with the subject in an environment. Some examples of personal sensors/devices that can monitor various aspects of a person's health include: fitness trackers (which may monitor heart rate, steps taken, calories burned, and/or sleep patterns); smartwatches (advanced ones can track heart rate, ECG, blood oxygen levels, and/or detect falls); wearable ECG monitors (to measure heart rate and rhythm, providing valuable data for those with heart conditions); pulse oximeters (to continuously monitor blood oxygen levels and pulse rate); continuous glucose monitors (CGMs, to track glucose levels in real-time, e.g. for people with diabetes); and wearable blood pressure monitors (to monitor blood pressure continuously).

Additionally, or alternatively, one of the plurality of sensors includes one or more ambient sensors associated with the environment. Some examples of sensors/devices that can be installed in an environment (e.g. home) to monitor activity, health, and overall wellbeing include: motion sensors (to detect movement within the environment, helping to monitor activity levels and detect falls or unusual inactivity); door sensors (to track when doors are opened or closed, providing insights into the person's movements and routines); smart cameras (cameras with motion detection and/or facial recognition can monitor activity and provide real-time video feeds, e.g. to caregivers); vital sign monitors (which can use radio-frequency technology to monitor heart rate, breathing, and other vital signs without the need for wearables); and environmental sensors (to monitor temperature, humidity, and/or air quality).

At a first step 310 of the method 300, sensor data and reference data for a first sensor of the plurality of sensors 202 is obtained. The sensor data is transmitted by the sensor (or device) to the computer implementing the method 300, for example. Initiating the method 300 may cause the computer to transmit a request to the sensor for its sensor data, e.g. recorded since the last validation check of the sensor, for example. The sensor data may thus be transmitted back to the computer in response to the request.

The reference data, for example, is based on a modelled normal operation of the first sensor, e.g. when monitoring the health of a given subject over a given time. The reference data may thus be associated with the subject, e.g. person, as well as the sensor used.

In some examples, the reference data comprises one or more pre-defined baseline values or ranges of values for the sensor data of the first sensor. The one or more pre-defined baseline values or ranges of values are indicative of the normal operation of the first sensor, for example.

At a second step 320, the sensor data is compared with the reference data. For example, it can be determined, using the reference data, whether the sensor data is indicative of anomalous operation of the first sensor. Thus, the method 300 may determine anomalous behaviour of the sensor by comparing the sensor data to the normal behaviour as detailed in the reference data, e.g. by comparing the sensor data to the baseline values or ranges of values.

In some examples, the method may include modelling normal behaviour of the sensor. The normal behaviour is modelled using the recorded sensor data, for example. A machine learning model may be generated to learn the normal operation of the sensor, for example, based on past sensor readings. The method may then determine anomalous behaviour of the sensor by comparing the operational sensor data to its modelled normal behaviour.

At a third step 330, a ticket 206 for the first sensor is generated based on a result of the comparison between the sensor data and reference data. The ticket 206 comprises an assigned status (e.g. pass or fail) for the sensor. In some embodiments, an anomaly score is assigned to the anomalous second device. The assigned status (or anomaly score) may be indicative of the severity of the anomalous sensor behaviour. The modelled normal behaviour of the sensor may include a plurality of threshold values for the anomaly score, each respectively corresponding to a different assignable status, for example.

At a fourth step 340, the assigned status is validated by comparing the sensor data to additional sensor data from a second sensor of the plurality of sensors 202. For example, the first and second sensors are different types of sensors employed to monitor the subject and/or environment. The respective sensor data of the two different sensors may be correlated. For example, the first sensor may be a heart rate monitor while the second sensor is a wearable electrocardiogram (ECG) monitor; both sensors configured to measure different but correlated heart activity data (e.g. heartrate vs electrogram data). In another example, the first sensor is a pedometer or activity tracker while the second sensor is a passive infrared (PIR) motion sensor. In this case, while the data measured by the two sensors are different types, they are correlated on a higher level, i.e. here they are correlated to movement activity of the subject. Increased activity of the subject measured by the first (pedometer) sensor can be correlated with the additional data about the movement of the subject from the second (PIR) sensor in the environment to see if the data agree/correspond.

The comparison of sensor data from different sensors may be done with a model, e.g. a mathematical or statistical model, or a machine learning model. The model may have different layers. For example, one layer may compare the same type of data output by different sensors of the same type, e.g. data values in the same or convertible units, that can be directly compared in the first order. Such as if a heart monitor and a smartwatch are compared, with both sensors outputting heart rate data with values in bpm. A higher layer of the model may be used/trained for when different types of sensors are compared but their outputs can be correlated, e.g. based on second-order outcomes/understandings. For example, if data from a step counter is compared to the output of PIR sensors, the number of steps a person takes between two PIR sensors in the environment can be measured and used to predict (i) if it is the individual, and (ii) if the step count is accurate, e.g. correlates with the pings from the two PIR sensors, such that they have arrived at the location that triggered the second PIR.

In examples, the validating is performed in response to the assigned status being indicative of the sensor data being anomalous based on the result of the comparison 320 with the reference data. For example, if it is determined from the comparison that the sensor data is anomalous based on its normal operation when monitoring the subject, a corresponding status (e.g. 'fail' or 'advisory') indicative of the anomaly is assigned to the first sensor as part of the sensor ticket 206 at step 330. The later step 340 of validating the assigned status based on additional data from a different sensor may thus be dependent on the status assigned to the first sensor, for instance only occurring when said status (e.g. 'fail' or 'advisory') is indicative of the sensor data measured by the first sensor being anomalous.

In other examples, the validation step 340 occurs regardless of the assigned status, e.g. as a "double check" for the sensor ticketing.

The validating 340 itself may involve changing the assigned status if the sensor data (for the first sensor) and additional sensor data (for the second sensor) do not correspond within a predetermined tolerance. On the other hand, if the sensor data and additional sensor data do correspond within the predetermined tolerance, the assigned status may be maintained.

At a fifth step 350, a certificate 210 of the first sensor is updated with the ticket 206 comprising the validated status of the first sensor. For example, the validated status, determined based on additional data from a second sensor, may be a different status to the originally assigned status determined based on reference data for the first sensor. Thus, the status recorded in the sensor ticket may be changed before the ticket is included in the certificate 210 of the sensor.

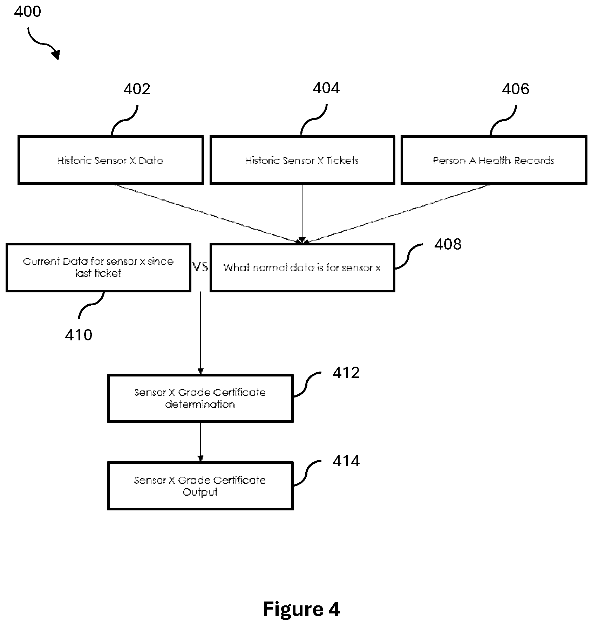

As mentioned, the reference data may be based on a modelled normal operation of the first sensor. For example, in the data processing workflow 400 shown in Figure 4, the modelled normal operation 408 for the first sensor ("sensor X") is modelled based on inputs including: historic sensor data 402 of sensor X; historic tickets 404 of sensor X; and an electronic health record 406 of the subject monitored by sensor X. In other examples, one or more of these inputs 402, 404, 406 may be used for the modelled normal operation 408 of the sensor.

In some examples, modelling the normal operation of the first sensor comprises training a machine learning model. Thus, the modelled normal operation 408 for the first sensor may comprise a trained machine learning model, e.g. classifier, which can classify sensor data as being anomalous (or normal).

In some embodiments, the machine learning model may be an unsupervised machine learning model. For example, the machine learning model may be generated using a k-means clustering algorithm. The cluster centres may be initialized using baseline values defined by the reference data. In other embodiments, the machine learning model may be a semi-supervised machine learning model. In such an embodiment, the machine learning model may use the reference data, e.g. past sensor data, as labelled training data the operational sensor data may include unlabelled training data. In yet further embodiments, the machine learning model may be a supervised model. In such an embodiment, labelled training data is used for training the machine learning model.

New sensor data 410 for sensor X since the last ticket is then compared with the modelled normal data 408 for that individual sensor. This comparison is then used to determine the overall grade certification 412 for the sensor X, which is then outputted 414 at the end of the workflow 400.

In some examples, the comparing of sensor data with reference data for a given sensor is used to determine whether the sensor data is anomalous due to sensor error. In such cases, the present computer-implemented methods may cause a request for intervention or corrective action to be output, e.g. by a user interface of the computer or sent to another device in the network. For example, corrective actions may include: resetting, by the computer/system, a connection of the sensor to the network; rebooting the sensor; performing a soft reset of the sensor (i.e. resetting the device without a loss of data); performing a hard reset of the sensor (i.e. resetting the device with a loss of all data); and/or executing any other computer-readable instructions to remedy the anomalous operation of the sensor.

Returning to Figure 3, the validating step 340 of the method 300 may thus be performed in response to determining that the sensor data is anomalous due not to sensor error. For example, if the sensor data is anomalous but not due to an inherent issue with the sensor, e.g. sensor degradation, it can be inferred that the anomaly is due to behaviour of the subject being monitored by the sensor.

In the alternative, determining that the sensor data is anomalous due to sensor error may cause further sensor data received from the first sensor to be excluded from further data analytics or monitoring of the plurality of sensors 202.

The validation method 300 may be iterated with a preset frequency. For example, the method is triggered, and sensor data obtained from the sensor, at regular time intervals. The sensor tickets 206 and/or sensor certificates 210 may include a frequency or time parameter for how often the respective sensor is checked/validated. The example sensor certificate 210 shown in Figure 2B includes a "frequency" parameter for the contact sensor having the value "24hrs". Thus, the validation method 300 is performed every 24 hours to check the sensor performance in this case. The sensor certificate 210 for the contact sensor in this example may thus be updated with a newly generated sensor ticket 206 every 24 hours.

In examples, the preset frequency with which the sensor is checked/validated may be modified in response to the assigned status being indicative of the sensor data being anomalous based on the result of the comparison with sensor data from other sensors. For example, a first (e.g. "pass") status may be assigned/validated if the sensor data agrees with the additional sensor data from other sensors, e.g. within a predetermined tolerance. A second (e.g. "fail") status may be assigned/validated if the sensor data is anomalous compared with the additional sensor data from other sensors, e.g. beyond a predetermined threshold of deviation. In some cases, a third (e.g. "advisory") status may be given to the sensor if the sensor data is anomalous, e.g. not within the tolerance for agreement, but is not so anomalous as to be beyond the deviation threshold. For example, there may be multiple thresholds/tolerances to set levels of deviation between "pass" and "fail".

In some examples, if the validated status of the sensor corresponds to such an intermediate (e.g. "advisory") status, the frequency parameter of the sensor ticket/certificate may be modified, e.g. increased such that the sensor is checked/validated more often, while in the intermediate state. In other words, the sensor ticket/certificate may have a given lifespan, e.g. between checks, which is shortened when the sensor is providing anomalous data and lengthened when the sensor data is consistent with data from other sensors. In certain cases, a predetermined number of checks may be performed with the sensor in the intermediate state before a "fail" status is assigned to the sensor by default.

As described, the check generally involves correlating the sensor data of different (types of) sensors to validate the readings from a given sensor to determine whether it can be trusted as giving valid readings.

For example, if a heart sensor routinely collects values between 40-60 bpm for a 90-year-old patient, it will be calculated/modelled using data analytics that this range of values is the 'normal' reading for the sensor. If the readings then suddenly increase to 190 bpm one day, e.g. far outside the normal range, it can be validated, e.g. checked whether the sensor is reading the values correctly. The process may first involve checking the new value(s) with the expected 'normal' to determine how deviated it is. In this example, the value has deviated significantly enough from the normal, e.g. beyond a predetermined threshold or tolerance, that it can be compared with another sensor in the environment to verify whether the value provided is true. If the values still do not match, e.g. the heart sensor data is anomalous compared to the other sensor data, it can be regarded as a faulty sensor, and a debugging process may be triggered. The sensor may then be removed from the data collection process until the sensor is manually checked or the values provided after a reset are consistent with the expected normal range for the sensor.

In some examples, however, if the sensor is a high priority sensor (such as a life support machine), the algorithm may stop using the values to check with the other sensors and instead prioritise the intervention, e.g. regardless of what the other sensors are reading. The priority level of a sensor may correspond to a weighting in a model for prioritising intervention, for example. Intervention may be prioritised between sensors/subjects based on a combination of sensor performance status and behavioural patterns monitored by said sensors. For example, if a sensor is working in the condition expected of it according to the certificate and it is raising an alert due to the results collected, this will be prioritised higher by the model. Whereas, if a sensor is not working as expected, the values would need to be verified with other sensors (where possible) to determine the priority of the intervention. In other words, the validation 340 of the status assigned to the sensor, using additional sensor data from one or more other sensors, is done in dependence of the priority level of the sensor. For example, a priority level for a given sensor may be represented by a priority value (or weighting) or a ranking among the plurality of sensors. This enables the condition of a sensor to be thoroughly verified and reduces the chance of false alerts being raised.

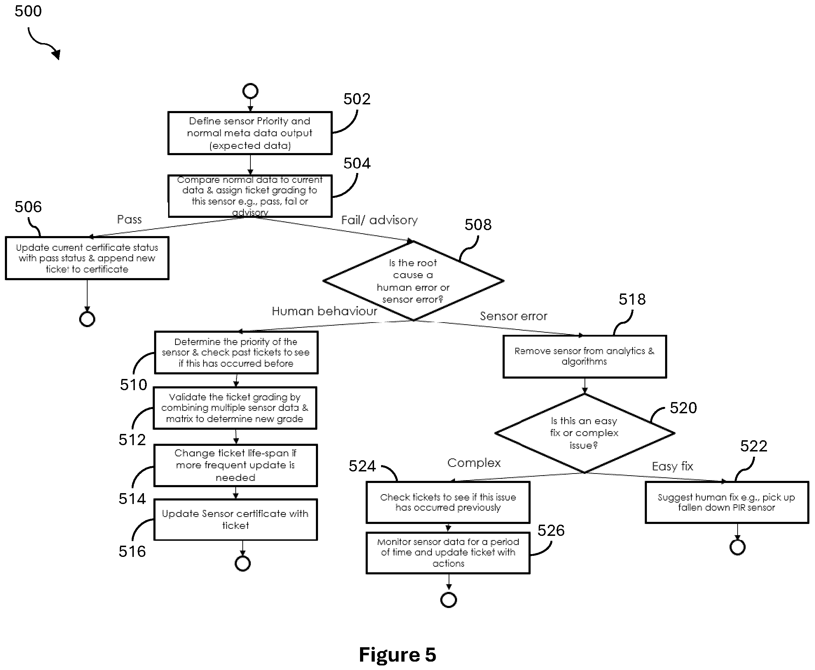

Figure 5 shows a flowchart for a method 500 according to a specific detailed embodiment of the present sensor validation mechanism, with multiple logic paths and outcomes.

Initially, at step 502, reference data (e.g. 'normal' meta data or expected data output) for a given sensor in the network is obtained. As mentioned, the reference data may be defined using historic sensor data, historic sensor tickets and an individual's health records. These data inputs are aggregated to define "normal" for the given sensor monitoring the given subject. The "normal" may be defined differently by a model, e.g. per the situation, using weightings. For example, different dimensions used would give confidence to the output. Historic sensor tickets offer more quantitative data whereas health records may be more qualitative, for example. Thus, contextual information can be provided to the model to determine the "normal" or expected value or range of values for the senor data.

Electronic health records for the subject may highlight certain characteristics about the subject. If a monitored patient has mobility issues, for example, they should not be able to scale a standard flight of stairs in < 10 seconds, so if the sensor data indicates this occurring, it can be determined that the sensor is not working as expected or the monitored subject has changed (e.g. someone else is now wearing the activity tracker).

In another example, an EHR for a pressure detection sensor (e.g. located on a factory line) may indicate the sensor historically has overheating issues. Thus, when the same type of pressure sensor is installed at a different location in the factory, the associated sensor tickets and/or EHR can be checked to ensure the values (or errors) outputted do not have the same context as the previous errors/alerts from the old sensor(s). If, however, no previous tickets or contextual data are associated with this new sensor, then no previous data may be used in the process of formulating the "normal behaviour" for the sensor. That is to say, the modelling of the "normal" behaviour of the new sensor starts from scratch.

A priority level for the sensor, amongst the plurality of sensors, is also defined at the initial step 502 of the method 500. For example, the plurality of sensors may be ranked in priority order. The priority level of a sensor may correspond to a weighting in a model for prioritising intervention, for example. In an implementation involving two patients, the first patient may have movement issues as part of their health record whereas the second patient has heart issues. Thus, the same sensors may be prioritised, e.g. weighted, differently in each case based on their importance to the relevant health issue(s). An example set of sensors and weightings for each of the two patients may be:
- Patient 1 (movement concern): PIR (0.5); pedometer (0.3); contact sensors (0.15); heart rate monitor (0.05).
- Patient 2 (heart issues): pacemaker (0.6); heart rate monitor (0.2); PIR (0.1); electricity disaggregation monitor (0.08); contact sensors (0.02).

For example, the PIR sensor(s) are weighted more than a heart monitor sensor for Patient 1 because their movement is the greatest concern. However, the opposite is true for Patient 2, where the sensors relating to the heart are weighted the most.

Next, at step 504, the defined normal meta data output of the sensor monitoring a given subject, e.g. an individual, is compared against the current data output of the sensor. This comparison may be run for one or more, e.g. each, sensor of the plurality of sensors within an environment.

A ticket is then generated based on the comparison. For example, a ticket may have different grade classifications (e.g. pass, fail or advisory). If the ticket state is indicative of the current sensor data being in agreement with the reference data for the given sensor, e.g. "pass", then the certificate status for the sensor can be updated with the pass status and the generated ticket appended to the sensor certificate (step 506). The method 500 is then ended as the sensor has passed the threshold requirements and determined to be in a satisfactory state.

If, on the other hand, the ticket state is indicative of the current sensor data being anomalous, i.e. not in agreement, with the reference data for the given sensor (e.g. "advisory" or "fail") then further steps of the method 500 are carried out. At step 508, it is checked whether the root cause of the anomaly/issue is due to a faulty sensor or subject, e.g. human, behaviour.

Optionally, if the root cause of the anomaly is due to subject behaviour (step 510), the priority of the sensor is determined, e.g. checked based on prerequisite information. For example, if it is a high priority sensor, the algorithm can disregard the fact the sensor is going wrong and attempt fix/intervene protocols. Past tickets can also be checked to see if a similar issue with the sensor has occurred before. This might allow for the system to try previous ticket fixes.

At step 512, the ticket grading for the sensor is validated with other sensors. For example, if activity data from a wearable activity tracker is showing increased movement for the individual, the output data of a motion sensor in the environment can validate the activity data, i.e. the increase in movement within the environment. This validation helps to make sure that the sensor reading from a given sensor, e.g. device in the environment, is accurate thereby reducing false prioritisation of interventions.

At optional step 514, a validation frequency or ticket lifespan for the sensor is modified. This change may be dependent on the new ticket grade, e.g. validated status, for the sensor. For example, if the grade/status is indicative of the sensor data being anomalous with the data from other sensors, the sensor certificate is then checked more frequently. This may occur when the determined anomaly is beyond a first threshold but not a second threshold (e.g. an "advisory" not a "fail" state) in examples. This part of the process acts as a dynamic validation mechanism for the sensors.

In some cases, if the sensor values are not as expected when validated with other sensor data, a process of resetting the sensor and conducting recovery protocols is triggered. If it is checked that this is done but the values outputted are still not as expected, then a request for human intervention may be triggered.

At step 516, the sensor certificate for the validated sensor is updated with the new ticket that has the validated ticket grading, i.e. sensor status, and the method 500 ends.

Optionally, if it is determined (step 508) that the sensor is faulty and is the root cause of the ticket failure, further steps 518-526 may be taken.

For example, the faulty sensor may be removed from the analytics algorithm (step 518), and/or the network of sensors, e.g. to prevent it skewing the data for validating the other sensors. In other words, if the sensor believes it has failed or it is reporting an error, its outputted results are not considered.

As previously mentioned, if the sensor initially reports a fail or advisory status (e.g. its readings are anomalous), it goes through the process of verifying 508 whether the result is sensor driven (failing sensor) or behaviour driven (subject doing something). If it is clarified that the subject is indeed doing something and the values are thus consistent/expected, the sensor ticket then falls into a pass (e.g. its readings are not considered anomalous) or advisory (e.g. its readings are anomalous but not to a degree corresponding with the sensor being in a fail state). However, if the anomalous readings are not verifiable using the other sensors, then other factors such as a priority of the sensor may impact the intervention/resolution.

At step 520, it is determined if the issue can be easily fixed by the user or is something more complex. If it can be fixed by the user (step 522) the fix is suggested by the system and checks on the sensor may be re-run. For example, if a PIR sensor has fallen over then a request to the user to pick it up and re-locate it to its original location may be triggered. Similarly, if a contact sensor fails, a request to check the sensor is connected properly may be triggered, e.g. if it is determined that the issue is fixable by the user. If it is a more complex sensor, though, such as an electricity disaggregation sensor or a life support machine, professional intervention may be requested/triggered.

At step 524, similarly to step 510, past tickets may be checked to see if a similar issue with the sensor has occurred before and if it could be fixed using stored knowledge.

After the issue is fixed, there may be a period of monitoring and testing the sensor based on the device information (step 526) before the method 500 ends. The ticket may also be updated with information about the actions taken for future reference, e.g. describing which solution solved the issue, to help prevent similar issues in the future or resolve them faster.

In the above embodiments, the first sensor is validated against the sensor data from a second sensor of the plurality of sensors. However, it is envisaged that the first sensor may be validated by comparing its sensor data with additional sensor data from multiple other sensors in the plurality of sensors. For examples, the additional sensor data may be aggregated, averaged, or otherwise pre-processed for comparing with the sensor data of the first sensor being validated.

For example, the network may have an arrangement of multiple sensors (or devices) in an IoT ecosystem, and it will be appreciated that the number of sensors/devices may vary. Specially, in certain embodiments, the network comprises multiple sensors/devices communicatively coupled together, wherein at least one of the sensors/devices comprises an IoT device.

Although the foregoing description has focussed on the monitoring of a subject in a medical context (i.e. in a context in which the subject is a human), it is also envisaged that the invention could equally be employed to monitor the health of a machine. For example, the environment may be a factory or warehouse, and the subject may be an industrial machine. Such a machine may be static or mobile (e.g. an autonomous vehicle or drone). In such cases, the "personal sensors" may be sensors associated with the machine (e.g. embedded sensors within the machine) for monitoring the machine's operation. Similarly, "health issues" in such cases may correspond to a fault condition for the machine in which the machine is no longer able to operate normally. It will be appreciated that, as in the medical context discussed above, many different types of fault condition may exist for a machine and similar techniques to those discussed above (e.g. the use of anomaly-based determinations) may be used to detect occurrences of each type of fault condition. In such cases, a monitoring system operating according to the invention may be deployed to monitor the machine by collecting contextual data from the available sensors (e.g. embedded and/or environmental sensors).

The preceding description is presented to enable any person skilled in the art to make and use the systems and/or perform the methods of the invention and is provided in the context of a particular application. Various modifications to the disclosed examples will be readily apparent to those skilled in the art. It is intended that the specification be considered as exemplary only.

Where this application lists one or more method steps, the presence of precursor, follow-on and intervening method steps is not excluded unless such exclusion is explicitly indicated. Similarly, where this application lists one or more components of a device or system, the presence of additional components, whether separate or intervening, is not excluded unless such exclusion is explicitly indicated.

In addition, where this application has listed the steps of a method or procedure in a specific order, it could be possible, or even expedient in certain circumstances, to change the order in which some steps are performed, and it is intended that the particular steps of the method or procedure claims set forth herein not be construed as being order-specific unless such order specificity is expressly stated in the claim. That is, the operations/steps may be performed in any order, unless otherwise specified, and embodiments may include additional or fewer operations/steps than those disclosed herein. It is further contemplated that executing or performing a particular operation/step before, partially or entirely contemporaneously with, or after another operation is in accordance with the described embodiments. For example, steps presented as occurring in series can, given appropriate means (e.g. parallel processors), be implemented in parallel, and vice-versa.

Insofar as embodiments of the invention described are implementable, at least in part, using a software-controlled programmable processing device, such as a microprocessor, digital signal processor or other processing device, data processing apparatus or system, it will be appreciated that a computer program for configuring a programmable device, apparatus or system to implement the foregoing described methods is envisaged as an aspect of the present invention. Such a computer program may be embodied as source code or undergo compilation for implementation on a processing device, apparatus or system or may be embodied as object code, for example.

Such a computer program may be encoded as executable instructions embodied in a carrier medium, non-transitory computer-readable storage device and/or a memory device in machine or device readable form, for example in volatile memory, non-volatile memory, solid-state memory, magnetic memory such as disk or tape, optically or magneto-optically readable memory such as magnetic tape, compact disk (CD), digital versatile disk (DVD) or other media that are capable of storing code and/or data. Such a computer program may alternatively or additionally be supplied from a remote source embodied in a communications medium such as an electronic signal, radio frequency carrier wave or optical carrier wave. Such carrier media are also envisaged as aspects of the present invention.

Such instructions, when executed by a processor (or one or more computers, processors, and/or other devices) may cause the processor (the one or more computers, processors, and/or other devices) to perform at least a portion of the methods described herein.

Where a processor is referred to herein, this is to be understood to refer to a single processor or multiple processors operably connected to one another. Similarly, where a memory is referred to herein, this is to be understood to refer to a single memory or multiple memories operably connected to one another.

The methods and processes can also be partially or fully embodied in hardware modules or apparatuses or firmware, so that when the hardware modules or apparatuses are activated, they perform the associated methods and processes. The methods and processes can be embodied using a combination of code, data, and hardware modules or apparatuses.

## Claims

1. A computer-implemented method of validating a sensor of a plurality of sensors monitoring the health of a subject, the method comprising:
obtaining sensor data and reference data for a first sensor of the plurality of sensors;
comparing the sensor data with the reference data;
generating a ticket, comprising an assigned status, for the first sensor based on a result of the comparing;
validating the assigned status by comparing the sensor data to additional sensor data from a second sensor of the plurality of sensors; and
updating a certificate of the first sensor with the ticket comprising the validated status of the first sensor.

2. The computer-implemented method of claim 1, wherein the plurality of sensors comprises:
one or more personal sensors associated with the subject in an environment; and/or
one or more ambient sensors associated with the environment.

3. The computer-implemented method of claim 1 or 2, wherein the first and second sensors are different types of sensors and the respective sensor data are correlated.

4. The computer-implemented method of any one of the preceding claims, wherein the validating is performed in response to the assigned status being indicative of the sensor data being anomalous based on the result of the comparing with the reference data.

5. The computer-implemented method of any one of the preceding claims, comprising determining, based on the comparing of the sensor data with the reference data, whether the sensor data is anomalous due to sensor error.

6. The computer-implemented method of claim 5, wherein the validating is performed in response to determining that the sensor data is anomalous due not to sensor error.

7. The computer-implemented method of claim 5, comprising excluding, in response to determining that the sensor data is anomalous due to sensor error, further sensor data received from the first sensor.

8. The computer-implemented method of any one of the preceding claims, wherein the reference data is based on a modelled normal operation of the first sensor.

9. The computer-implemented method of claim 8, wherein the modelled normal operation is modelled based on inputs including one or more of: historic sensor data of the first sensor; historic tickets of the first sensor; and an electronic health record of the subject monitored by the first sensor.

10. The computer-implemented method of any one of the preceding claims, wherein the validating comprises:
changing the assigned status if the sensor data for the first sensor and the additional sensor data for the second sensor do not correspond within a predetermined tolerance based on the comparing; or
maintaining the assigned status if the sensor data for the first sensor and the additional sensor data for the second sensor correspond within a predetermined tolerance based on the comparing.

11. The computer-implemented method of any one of the preceding claims, comprising iterating the method one or more times with a preset frequency.

12. The computer-implemented method of claim 11, comprising modifying the preset frequency in response to the assigned status being indicative of the sensor data being anomalous based on the result of the comparing.

13. The computer-implemented method of any one of the preceding claims, wherein the validating is performed based on a priority level of the first sensor relative to the plurality of sensors.

14. A data processing system comprising a processor configured to perform the method of any one of the preceding claims.

15. A computer program comprising instructions which, when the program is executed by a computer, cause the computer to carry out the method of any one of claims 1 to 13.
